# EUROPÄISCHE PATENTANMELDUNG

(11) **EP 1 801 584 A1**
(43) Veröffentlichungstag der Anmeldung: **27.06.2007**
(21) Anmeldenummer: 05028456.1
(22) Anmeldetag: 24.12.2005
(51) Int. Cl.: G01N 33/487

(54) **Analysesystem für Körperflüssigkeiten**

(71) Anmelder: F.HOFFMANN-LA ROCHE AG, 4070 Basel (CH); Roche Diagnostics GmbH, 68305 Mannheim (DE)
(72) Erfinder: List, Hans, 64754 Hesseneck-Kailbach (DE); Krämer, Uwe, 68549 Livesheim (DE); Hess, Peter, 68165 Mannheim (DE)
(74) Vertreter: Pfiz, Thomas

(57) **Zusammenfassung**

Die vorliegende Erfindung betrifft ein Analysesystem für Körperflüssigkeiten, insbesondere tragbares Blutzuckermessgerät (10), mit einem Testband (23) zur Applikation der zu analysierenden Körperflüssigkeit. Erfindungsgemäß ist vorgesehen, dass das Testband (23) Schlaufenbildungsmittel aufweist, die eine Bildung einer der Applikation der zu analysierenden Körperflüssigkeit dienenden Bandschlaufe (30) nach Freigabe eines Bandabschnitts (35) bewirken.

## Beschreibung

Die Erfindung betrifft ein Analysesystem für Körperflüssigkeiten, insbesondere tragbares Blutzuckermessgerät, mit einem Testband zur Applikation der zu analysierenden Körperflüssigkeit.

Ein gattungsgemäßes Analysesystem in Form eines tragbaren Blutzuckermessgeräts ist in der WO 2005/006985 A2 beschrieben. Dieses Analysesystem arbeitet gleichsam als Minilabor und ermöglicht regelmäßige Blutzuckerkontrollen, die für Diabetiker unerlässlich sind. Die hierzu erforderlichen Schritte sollen auch von Laien einfach und schnell vorgenommen werden können. Daher wird ein weitgehend automatisierter Messablauf angestrebt. Hierfür wird bei dem bekannten Analysesystem jeweils ein mit geeigneten Trockenchemikalien versehener Testbandabschnitt in einem Probeaufnahmebereich freigelegt, so dass Körperflüssigkeit gezielt appliziert werden kann. Für die Analyse wird der mit Körperflüssigkeit beaufschlagte Testbandabschnitt dann beispielsweise einer optischen Messeinheit zugeführt.

Die Freilegung des Testbandabschnitts geschieht dadurch, dass ein Teil des Testbands mittels mechanischer Mittel von einer Vorratsrolle abgezogen wird, so dass der mit den Trockenchemikalien versehene Testbandabschnitt, der permanent unter Zugspannung steht, von außen zugänglich wird. Die hierfür erforderlichen mechanischen Mittel sind aber nur aufwändig und daher kostenintensiv zu realisieren, da sie aufgrund des geringen Platzangebots sowohl in ihrer konkreten Ausgestaltung als auch in ihrer Positionierung innerhalb des Analysesystems relativ engen Limitationen unterliegen. Außerdem wird mit dem Testbandabschnitt auch zwangsläufig ein mechanisches Bauteil in den Probeaufnahmebereich verbracht, was die Gefahr der Verschmutzung von mechanischen Bauteilen erhöht. Schließlich ist es für den Benutzer eines solchen Analysesystems insbesondere in Stresssituationen und bei gesundheitlicher Beeinträchtigung nicht immer einfach, die Körperflüssigkeit gezielt auf dem relativ kleinen Testbandabschnitt zu applizieren.

Die Aufgabe der vorliegenden Erfindung besteht somit darin, ein einfach zu bedienendes Analysesystem in herstellungstechnisch günstiger Weise bereit zu stellen.

Zur Lösung dieser Aufgabe wird die im unabhängigen Anspruch angegebene Merkmalskombination vorgeschlagen. Vorteilhafte Ausgestaltungen und Weiterbildungen der Erfindung ergeben sich aus den abhängigen Ansprüchen.

Demnach wird vorgeschlagen, dass das Testband Schlaufenbildungsmittel aufweist, die eine Bildung einer der Applikation der zu analysierenden Körperflüssigkeit dienenden Bandschlaufe nach Freigabe eines Bandabschnitts bewirken. Es ist also nicht mehr notwendig, ein mechanisches Bauteil in den Probenaufnahmebereich, d.h. in den Bereich der Applikation der Körperflüssigkeit zu verbringen, da sich durch die erfindungsgemäßen Maßnahmen selbsttätig eine Bandschlaufe bildet. Hierfür muss lediglich der zur Bildung der Bandschlaufe vorgesehene Bandabschnitt freigegeben sein. Das bedeutet, dass entweder die bei der Bildung der Bandschlaufe wirkende Kraft größer ist als die Bandzugkraft, die das Testband gespannt hält oder dass die Bandzugkraft in dem zur Bildung der Bandschlaufe vorgesehenen Bandabschnitt reduziert oder aufgehoben ist. Die hierfür notwendigen Mittel können ausnahmslos im Inneren des erfindungsgemäßen Analysesystems angeordnet sein und sind nicht daher nicht mehr von außen zugänglich. Damit wird die Gefahr der Verschmutzung mechanischer Bauteile vermieden. Die selbsttätig gebildete Bandschlaufe kann je nach Ausgestaltung des erfindungsgemäßen Analysesystems unterschiedlich groß gewählt werden. Lediglich die Größe der mit den Trockenchemikalien versehenen Testbereiche ist an die Größe der gebildeten Bandschlaufe anzupassen, so dass immer eine für die Applikation von Körperflüssigkeit geeignete Bandschlaufe zur Verfügung steht. Eine große Bandschlaufe mit einem entsprechend großen mit Trockenchemikalien versehenen Testbereich ermöglicht es dem Benutzer des erfindungsgemäßen Analysesystems auch in Stresssituationen oder bei gesundheitlicher Beeinträchtigung, die Körperflüssigkeit gezielt auf der Bandschlaufe zu applizieren.

Gemäß einer vorteilhaften Ausführung weist das Testband zumindest abschnittsweise mit Schlaufenbildungsmitteln versehene Testbereiche zur Applikation der zu analysierenden Körperflüssigkeit auf. Das Testband ist in diesem Fall mit einer Vielzahl fortlaufender diskreter Testbereiche versehen, die jeweils mit Schlaufenbildungsmitteln ausgestattet sind. Es wäre aber auch denkbar, das Testband mit einem ununterbrochenen, mit durchgängigen Schlaufenbildungsmitteln ausgestatteten Testbereich zu versehen.

Das Schlaufenbildungsmittel kann so ausgestaltet sein, dass es nach Freigabe eines Bandabschnitts unmittelbar, das heißt, ohne zusätzliche Einwirkung von außen, die Bildung einer Bandschlaufe bewirkt. Diese besonders einfache und kostengünstige Ausführungsform wird bevorzugt dadurch realisiert, dass das Testband einen vorzugsweise durch.etikettenartig aufgebrachte Testfelder gebildeten Schichtaufbau aufweist, welcher durch schichtweise unterschiedliche Vorspannungen nach Freigabe eines Bandabschnitts unmittelbar eine Bandschlaufe bildet.

Eine vorteilhafte Ausführung sieht vor, dass dem Testband in entspanntem Zustand Auswölbungen eingeprägt sind. Im entspannten Zustand, wie er im Bereich des freigegebenen Bandabschnitts gegeben ist, nimmt das Testband also von selbst eine gewölbte Lage ein. Diese Wölbung kann beispielsweise durch auf oder in dem Testband vorgesehene Federelemente stabilisiert oder verstärkt sein, so dass sich nach Freigabe eines Bandabschnitts besonders zuverlässig eine Bandschlaufe bildet.

In einer anderen Ausgestaltung des erfindungsgemäßen Analysesystem kann zusätzlich ein Aktivierungsmittel vorgesehen sein, das von außen auf das Schlaufenbildungsmittel einwirkt. Das Schlaufenbildungsmittel wird also entweder gleichzeitig mit der Freigabe des Bandabschnitts oder danach gezielt aktiviert, so dass sich die Bandschlaufe erst mit der Aktivierung, also vom Benutzer kontrolliert, bildet. Das Aktivierungsmittel wirkt dabei in demjenigen räumlichen Bereich des erfindungsgemäßen Analysesystems, in dem sich die Bandschlaufe bilden soll.

Es gibt zahlreiche Möglichkeiten, das Testband mit durch äußere Einwirkung gezielt aktivierbaren Schlaufenbildungsmitteln zu versehen. Das Testband kann beispielsweise mit als Schlaufenbildungsmittel dienenden magnetischen Partikeln und/oder magnetischen Beschichtungen versehen sein. Insbesondere kann auf dem Testband eine Kleberschicht aufgetragen sein, die magnetisches Material in Form von feinem Pulver enthält. Das Testband kann auch mit einer dünnen Folie aus magnetischem Material laminiert sein. Als Aktivierungsmittel dient dann ein einfacher Magnet, der das magnetische Material unter Bildung einer Bandschlaufe anzieht.

Auch piezoelektrisch verformbare Elemente sind als Schlaufenbildungsmittel geeignet. Als Aktivierungsmittel ist dann eine Spannungsquelle vorgesehen ist, die die piezoelektrisch verformbaren Elemente unter Bildung einer Bandschlaufe verformt. So gibt es Polymerfolien mit piezoelektrischen Eigenschaften, mit denen das Testband beispielsweise unterlegt sein kann. Wenn eine Spannung zwischen den beiden Seiten der Polymerfolie angelegt wird, wölbt sich das Testband unter Bildung einer Bandschlaufe nach vorne.

Das Testband kann auch mit Formgedächtnis-Elementen versehen sein, die mittels einer Energiequelle, insbesondere für elektrische Energie, magnetische Energie oder Strahlungsenergie, aktivierbar sind. Im Testband können beispielsweise Einlagen aus einer Formgedächtnis-Legierung eingearbeitet sein. Wenn der betreffende Bandabschnitt über die Umwandlungstemperatur hinaus erwärmt wird, verformt er sich zu der gewünschten Bandschlaufe. Die Erwärmung kann mittels direkter oder induktiver Stromdurchleitung oder durch Eintrag von Strahlungsenergie, beispielsweise Mikrowellenstrahlung, erfolgen. Die Umwandlungstemperaturen von Formgedächtnis-Legierungen lassen sich durch die Materialzusammensetzung über einen weiten Temperaturbereich einstellen, bei Nickel-Titan-Legierungen beispielsweise von -15° bis +80°C.

Anstelle von Elementen aus Formgedächtnis-Legierungen können auch Elemente in Form von Bimetallstreifen verwendet werden, die auf die gleiche Art und Weise aktiviert werden wie Formgedächtnis-Legierungen.

Als Vorrichtung zur Freigabe eines Bandabschnitts dient in einer bevorzugten Ausführungsform ein feststellbarer und auslenkbarer Umlenkhebel, der in der Feststellposition die Auslenkung des Bandabschnitts und in der ausgelenkten Position die Freigabe des Bandabschnitts bewirkt. Damit ist eine besonders einfache und kompakte Bauweise möglich, so dass der Umlenkhebel vergleichsweise problemlos vollständig in das erfindungsgemäße Analysesystem integriert werden kann, so dass er von außen nicht zugänglich ist. Der Umlenkhebel ist vorzugsweise gegen eine Federkraft auslenkbar, kann aber auch stattdessen oder zusätzlich motorbetrieben sein.

Wenn der Umlenkhebel um einen konstanten Betrag auslenkbar ist, beispielsweise indem ein Anschlagelement die Auslenkung begrenzt, werden Bandabschnitte konstanter Länge freigegeben werden, was eine regelmäßige Anordnung der Testbereiche auf dem Testband erlaubt.

Wenn das Testband auf Wickelspulen zur Aufnahme frischer bzw. verbrauchter Testbandbereiche aufgespult ist, ist der Umlenkhebel bevorzugt im Bereich der Wickelspule zur Aufnahme verbrauchter Testbandbereiche angeordnet.

Das Testband kann in eine Bandkassette integriert sein, so dass ein vollständig aufgebrauchtes Testband vom Benutzer auf einfache Weise ausgetauscht werden kann. In diesem Fall ist die Vorrichtung zur Freigabe eines Bandabschnitts zweckmäßigerweise in die Bandkassette integriert.

Im Folgenden wird die Erfindung anhand des in der Zeichnung schematisch dargestellten Ausführungsbeispiels näher erläutert. Es zeigen
- Fig. 1: eine perspektivische, teilweise Darstellung eines tragbaren Blutzuckermessgeräts in einer Ausgangsstellung;
- Fig. 2: das Blutzuckermessgerät gemäß Figur 1, mit einer ausgebildeten Bandschlaufe zur Applikation eines Bluttropfens;
- Fig. 3: das Blutzuckermessgerät gemäß Figur 1 in einer Messstellung.

Das in der Zeichnung teilweise dargestellte Blutzuckermessgerät 10 als beispielhafte Ausführungsform eines erfindungsgemäßen Analysesystems besteht im Wesentlichen aus einem in der Zeichnung lediglich angedeuteten Gehäuse 11, einem daran angebrachten Blutentnahmekonus 12 als Aufnahme für einen Finger eines Probanden, einer in der Zeichnung ebenfalls nur angedeuteten, im Gehäuse 11 angeordneten Stecheinheit 13 mit einem Stechorgan 14 zum Einstechen in den im Blutentnahmekonus 12 aufgenommenen Finger und einer Bandeinheit 20.

Die Bandeinheit 20 ist als Bandkassette mit einer äußeren Gehäuseschale 21 und einer inneren Gehäuseschale 22 dargestellt und nimmt ein Testband 23 auf. Das Testband 23 weist eine Vielzahl fortlaufender, diskreter Testbereiche 24 auf, die mit Trockenchemikalien beschichtet sind, welche mit dem darauf aufgebrachten Blut reagieren und dabei eine der Blutzuckerkonzentration entsprechende, optisch detektierbare Farbänderung bewirken. Das Testband 23 ist auf Wickelspulen aufgewickelt. Es ist im konkreten Fall von einer Vorratsrolle 25 abziehbar und auf eine Aufwickelrolle 26 aufspulbar. Hierfür ist an der Aufwickelrolle 26 ein Bandvorschubantrieb vorgesehen (nicht dargestellt). Die Vorratsrolle 25 ist nicht angetrieben, sondern lediglich gebremst.

Das von der Vorratsrolle 25 abgezogene Testband 23 ist zwischen der äußeren Gehäuseschale 21 und der inneren Gehäuseschale 22 geführt, mit Ausnahme eines Gehäuseabschnitts 27 im Bereich des Blutentnahmekonus 12, wo die äußere Gehäuseschale 21 unterbrochen ist. In diesem Abschnitt 27 ist das Testband 23 durch den Blutentnahmekonus 12 hindurch zugänglich.

Die innere Gehäuseschale 22 trennt eine Kavität 28 ab, in der die Vorratsrolle 25 aufgenommen ist, so dass das darauf aufgewickelte Testband 23 vor Kontamination geschützt ist. Eine Dichtung 29 ist in Transportrichtung des Testbandes 23 vor dem Abschnitt 27 an der äußeren und inneren Gehäuseschale 21, 22 vorgesehen und verhindert, dass von dem von außen zugänglichen Abschnitt 27 des Testbandes 23 aus Fremdstoffe in die Kavität 28 eindringen und das auf der Vorratsrolle 25 aufgewickelte Testband 23 kontaminieren.

Die innere Gehäuseschale 22 begrenzt ferner einen Messraum 31, in dem eine Messeinrichtung zur optischen Detektion der Blutzuckerkonzentration aufgenommen ist (nicht dargestellt). In diesem Bereich weist die innere Gehäuseschale ein Messfenster 32 auf.

Das von der Vorratsrolle 25 abgezogene Testband 23 ist vor der Aufwickelrolle 26 über eine erste Umlenkrolle 33 und eine zweite Umlenkrolle 34 geführt, so dass ein in etwa U-förmiger Bandabschnitt 35 gebildet ist. Die zweite Umlenkrolle 34 ist feststehend, wohingegen die erste Umlenkrolle 33 am freien Ende eines Umlenkhebels 36 drehbar gelagert ist, der wiederum auf der Achse der Aufwickelrolle 26 in Richtung des Pfeiles A schwenkbar gelagert ist. Der Umlenkhebel 36 liegt an einem Anschlagstift 37 an.

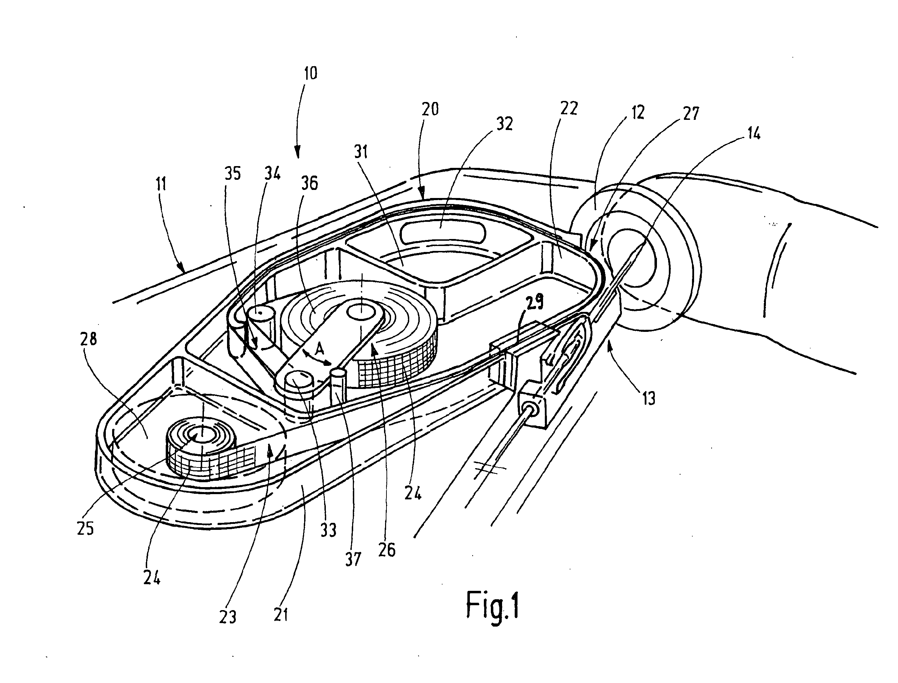

In der in Figur 1 dargestellten Ausgangsstellung ist der Umlenkhebel 36 maximal ausgeschwenkt, so dass er am Anschlagstift 37 anliegt und der U-förmige Bandabschnitt 35 gebildet ist. In dieser Position ist der Umlenkhebel 36 arretiert, beispielsweise mittels der Federkraft einer Rückstellfeder (nicht dargestellt) oder durch einen separaten Antriebsmechanismus in dieser Position gehalten. Nun kann mittels des Bandvorschubantriebs Testband 23 von der Vorratsrolle 25 abgezogen und auf die Aufwickelrolle 26 aufgespult werden. Der Bandvorschubantrieb wirkt dabei insbesondere gegen eine von der Dichtung 29 auf das Testband 23 ausgeübte Reibkraft, welche das Testband 23 innerhalb der Dichtung 29 festhält. In dieser Ausgangsstellung kann der Benutzer, der eine Blutzuckermessung durchführen will, einen Finger in den Blutentnahmekonus 12 eindrücken und die Stecheinheit 13 betätigen, so dass, wie dargestellt, das Stechelement 14 aus der Stecheinheit 13 ausfährt und in die Fingerbeere einsticht und ein Bluttropfen austritt.

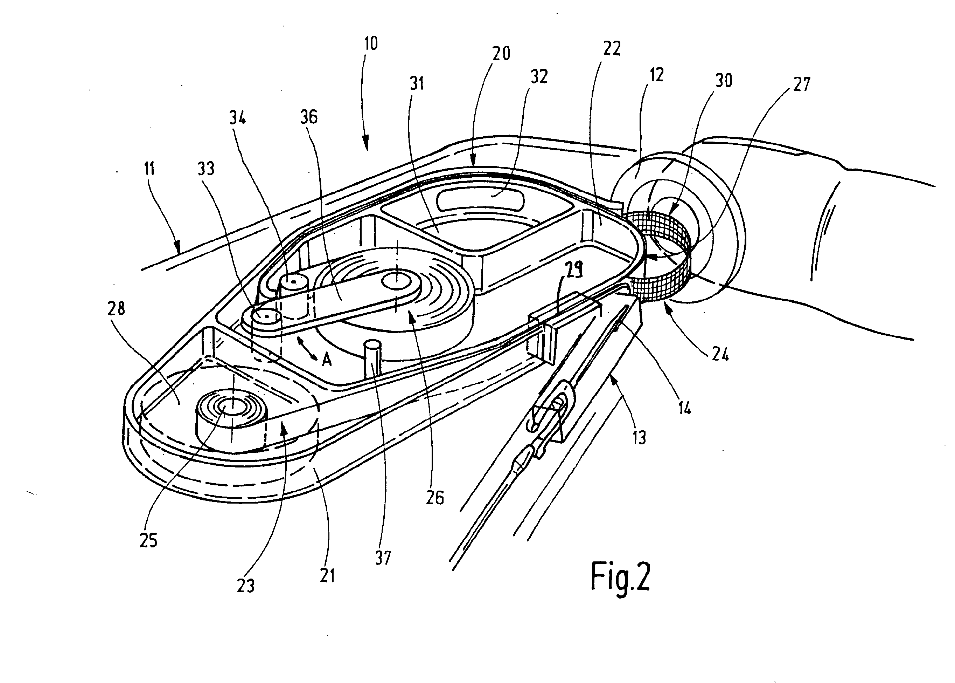

In Figur 2 ist der sich anschließende zweite Verfahrensschritt dargestellt. Das Stechelement 14 ist wieder in die Stecheinheit 13 eingefahren. Der Umlenkhebel 36 ist aus seiner Arretierung gelöst und in die in Figur 2 gezeigte Position geschwenkt, wobei er an der feststehenden Umlenkrolle 34 anliegt. Hierbei muss der Umlenkhebel 36 vom Benutzer aktiv gegen die Rückstellkraft der Rückstellfeder bewegt werden. Der U-förmige Bandabschnitt ist damit freigegeben. Dieser Bandabschnitt kann nicht auf die Aufwickelrolle 26 aufgespult werden, da deren Bandvorschubantrieb nicht betätigt wird. Dieser Bandabschnitt kann aber auf nicht auf die Vorratsrolle 25 aufgewickelt werden, weil diese gebremst ist und weil die von der Dichtung 29 auf das Testband 23 ausgeübte Reibkraft das Testband in der Dichtung 29 festhält. Daher ist in diesem freigegebenen Bandabschnitt nun die sonst auf das Testband 23 wirkende Zugspannung aufgehoben. Der Testbereich 24, der in dem von außen zugänglichen Abschnitt 27 unterhalb des Blutentnahmekonus 12 angeordnet sind, bildet nun selbsttätig eine Bandschlaufe 30, da er mit einem der bereits beschriebenen Schlaufenbildungsmittel ausgestattet ist. Die Bandschlaufe 30 bildet sich entweder spontan oder durch Betätigung eines Aktivierungsmittels. Nun kann der aus der Fingerbeere ausgetretene Bluttropfen auf die Bandschlaufe 30 und damit auf den mit Trockenchemikalien beschichteten Testbereich 24 appliziert werden.

In einer bevorzugten Ausführungsform mit integriertem Schlaufenbildungsmittel wird das Testband 23 so hergestellt, dass auf ein durchgehendes Trägerband aus Kunststoff einzelne Etiketten als Testbereich bzw. Testaufbau 24 aufgeklebt werden. Der Testaufbau besteht aus einer Kunststofffolie, die mit der Testchemie beschichtet ist. Darüber liegt noch ein Kunststoffgewebe.

Wird nun bei der Applikation der Testetiketten das Trägerband im elastischen Bereich gedehnt und nach der Applikation entlastet, so wird sich aufgrund der asymmetrischen Vorspannung im Testbereich 24 eine Krümmung einstellen, wobei die Trägerseite konkav ist und die Chemieseite konvex ist. Nach dem Aufwickeln des Testbandes 23 bleibt die Krümmung auch über lange Zeit erhalten, weil die Testfelder auf dem Bandwickel gekrümmt vorliegen. Erst beim Bandtransport im Gerät 10 werden die Testfelder kurzzeitig gerade gezogen. Die Tendenz zur Krümmung bei nachlassendem Bandzug bleibt aber erhalten. Diese Krizmmungsneigung kann ggf. noch verstärkt werden, indem das Band 23 beim Bandvorlauf über eine im Gerät 10 oder in der Kassette 20 eingebaute scharfe Umlenkkante gezogen wird.

Die Größe der gebildeten Bandschlaufe 30 hängt von der Größe des U-förmigen Bandabschnitts 35 ab, welche wiederum dadurch bestimmt ist, wie weit der Umlenkhebel 36 geschwenkt werden kann. Wenn die Bildung einer vergleichsweise großen Bandschlaufe 30 gewünscht ist, um das Applizieren des Bluttropfens zu vereinfachen, kann dies durch die konkrete Auslegung des Schwenkwegs des Umlenkhebels eingestellt werden. Zweckmäßigerweise ist der Testabschnitt 24 etwa so groß wie die gebildete Bandschlaufe 30, so dass der Bluttropfen in jedem Fall auf den Testabschnitt 24 appliziert wird.

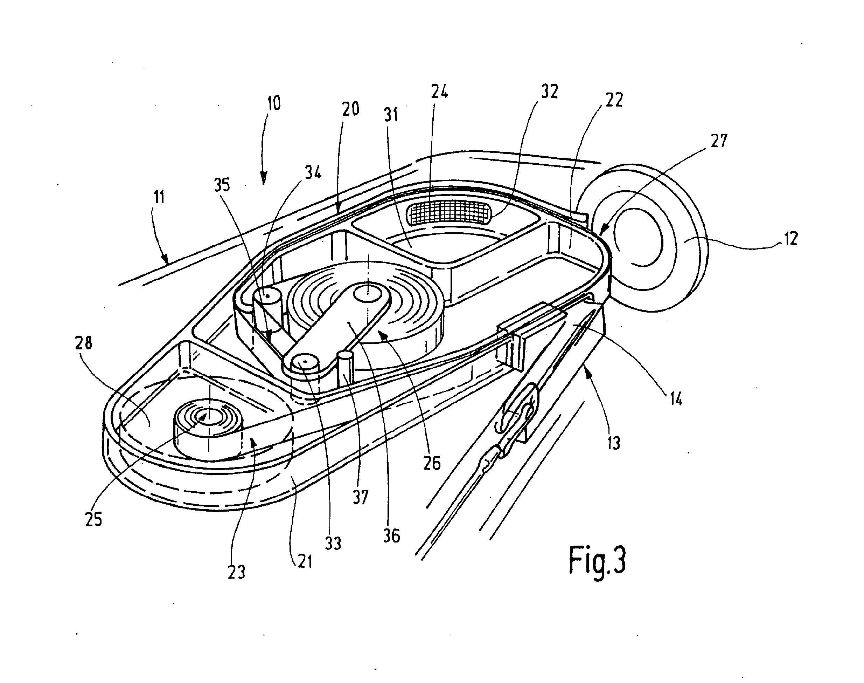

Figur 3 zeigt den sich daran anschließenden Verfahrensschritt. Nachdem der Benutzer die aktive Betätigung des Umlenkhebels 36 beendet hat, schwenkt dieser durch die Einwirkung der Rückstellfeder wieder in seine bereits in Figur 1 gezeigte Ausgangsstellung zurück, so dass er am Anschlagstift 37 anliegt. Dabei wird die Bandschlaufe wieder gespannt, so dass der mit dem Bluttropfen beaufschlagte Testabschnitt in Richtung des Messfensters 32 transportiert wird. Gleichzeitig wird wieder ein U-förmiger Bandabschnitt 35 gebildet. Durch den Transport des Testbands 23, das heißt, durch Aufspulen des Testbands 23 auf die Aufwickelrolle 26 und damit Abwickeln des Testbands 23 von der Vorratsrolle 25, wird der mit Blut beaufschlagte Testabschnitt 24 vor dem Messfenster 32 positioniert; so dass die im Messraum befindliche Messeinrichtung den Blutzuckergehalt messen kann.

Die Begrenzung der Schwenkbewegung des Umlenkhebels 36 durch den Anschlagstift 37 bewirkt, dass nach jedem Messvorgang immer gleich große U-förmige Bandabschnitte 35 gebildet werden. Daher können die mit Schlaufenbildungsmittel versehenen Testabschnitte 24 in regelmäßigen Abständen auf dem Testband 23 angeordnet werden. Die Größe der Abstände richtet sich dabei nach der Größe und Ausgestaltung des Blutzuckermessgeräts 10 und der Größe des U-förmigen Bandabschnitts 35.

Wenn eine neue Blutzuckermessung vorgenommen werden soll, wird der Bandvorschubantrieb der Aufwickelrolle 26 betätigt, so dass das Testband 23 so weit transportiert wird, dass wieder ein Testbereich im vom Blutentnahmekonus 12 aus zugänglichen Abschnitt 27 liegt und die in Figur 1 gezeigte Ausgangssituation gegeben ist.

## Patentansprüche

1. Analysesystem für Körperflüssigkeiten, insbesondere tragbares Blutzuckermessgerät (10), mit einem Testband (23) zur Applikation der zu analysierenden Körperflüssigkeit, **dadurch gekennzeichnet, dass** das Testband (23) Schlaufenbildungsmittel aufweist, die eine Bildung einer der Applikation der zu analysierenden Körperflüssigkeit dienenden Bandschlaufe (30) nach Freigabe eines Bandabschnitts (35) bewirken.

2. Analysesystem nach Anspruch 1, **dadurch gekennzeichnet, dass** das Testband (23) zumindest abschnittsweise mit Schlaufenbildungsmittel versehene Testbereiche (24) zur Applikation der zu analysierenden Körperflüssigkeit aufweist.

3. Analysesystem nach Anspruch 1 oder 2, **dadurch gekennzeichnet, dass** das Schlaufenbildungsmittel nach Freigabe eines Bandabschnitts (35) unmittelbar die Bildung einer Bandschlaufe (30) bewirkt.

4. Analysesystem nach einem der Ansprüche 1 bis 3, **dadurch gekennzeichnet, dass** das Testband (23) einen vorzugsweise durch etikettenartig aufgebrachte Testfelder (24) gebildeten Schichtaufbau aufweist, welcher durch schichtweise unterschiedliche Vorspannungen nach Freigabe eines Bandabschnitts (35) unmittelbar eine Bandschlaufe (30) bildet.

5. Analysesystem nach einem der Ansprüche 1 bis 4, **dadurch gekennzeichnet, dass** dem Testband (23) in entspanntem Zustand Auswölbungen eingeprägt sind, insbesondere durch auf oder in dem Testband (23) vorgesehene Federelemente, so dass sich nach Freigabe eines Bandabschnitts (35) unmittelbar eine Bandschlaufe (30) bildet.

6. Analysesystem nach Anspruch 1 oder 2, **dadurch gekennzeichnet, dass** ein Aktivierungsmittel vorgesehen ist, das das Schlaufenbildungsmittel aktiviert, so dass es die Bildung einer Bandschlaufe (30) bewirkt.

7. Analysesystem nach Anspruch 6, **dadurch gekennzeichnet, dass** das Testband (23) mit als Schlaufenbildungsmittel dienenden magnetischen Partikeln und/oder magnetischen Beschichtungen versehen ist und als Aktivierungsmittel ein Magnet vorgesehen ist.

8. Analysesystem nach Anspruch 6, **dadurch gekennzeichnet, dass** das Testband (23) mit als Schlaufenbildungsmittel dienenden piezoelektrisch verformbaren Elementen versehen ist und als Aktivierungsmittel eine Spannungsquelle vorgesehen ist.

9. Analysesystem nach Anspruch 6, **dadurch gekennzeichnet, dass** das Testband (23) mit als Schlaufenbildungsmittel dienenden Formgedächtnis-Elementen, insbesondere Formgedächtnis-Legierungen oder Bimetallstreifen, versehen ist und als Aktivierungsmittel eine Energiequelle, insbesondere für elektrische Energie, magnetische Energie oder Strahlungsenergie, vorgesehen ist.

10. Analysesystem nach einem der Ansprüche 1 bis 9, **dadurch gekennzeichnet, dass** als Vorrichtung zur Freigabe eines Bandabschnitts ein feststellbarer und auslenkbarer Umlenkhebel (36) vorgesehen ist, der in der Feststellposition die Auslenkung des Bandabschnitts (35) und in der ausgelenkten Position die Freigabe des Bandabschnitts (35) bewirkt.

11. Analysesystem nach Anspruch 10, **dadurch gekennzeichnet, dass** der Umlenkhebel (36) gegen eine Federkraft auslenkbar ist.

12. Analysesystem nach Anspruch 10 oder 11, **dadurch gekennzeichnet, dass** der Umlenkhebel (36) um einen konstanten Betrag auslenkbar ist, insbesondere mittels eines Anschlagelements (37), so dass Bandabschnitte (35) konstanter Länge freigegeben werden.

13. Analysesystem nach einem der Ansprüche 10 bis 12, **dadurch gekennzeichnet, dass** das Testband (23) auf Wickelspulen (25, 26) zur Aufnahme frischer bzw. verbrauchter Testbandbereiche aufgespult ist und der Umlenkhebel (36) im Bereich der Wickelspule (26) zur Aufnahme verbrauchter Testbandbereiche angeordnet ist.

14. Analysesystem nach einem der Ansprüche 1 bis 13, **dadurch gekennzeichnet, dass** das Testband (23) in eine Bandkassette integriert ist.

15. Analysesystem nach Anspruch 14, **dadurch gekennzeichnet, dass** die Vorrichtung (36) zur Freigabe eines Bandabschnitts (35) in die Bandkassette integriert ist.
